# EUROPÄISCHE PATENTSCHRIFT

(11) **EP 1 080 177 B1**
(45) Veröffentlichungstag und Bekanntmachung des Hinweises auf die Patenterteilung: **02.10.2002**
(21) Anmeldenummer: 99926419.5
(22) Anmeldetag: 26.05.1999
(51) Int. Cl.: C12M 1/00, C12N 1/00

(54) **NEUES VERFAHREN ZUM TRANSFER VON BIOLOGISCHEM MATERIAL**
NEW METHOD FOR TRANSFERRING BIOLOGICAL MATERIAL
NOUVEAU PROCEDE POUR LE TRANSFERT DE MATERIAU BIOLOGIQUE

(30) Priorität: 29.05.1998 DE 19824243
(43) Veröffentlichungstag der Anmeldung: 07.03.2001
(73) Patentinhaber: Deutsches Ressourcenzentrum für Genomforschung GmbH, 14059 Berlin (DE)
(72) Erfinder: BIENERT, Klaus, D-14550 Gross Kreutz (DE); KRAACK, Heiko, D-14469 Potsdam (DE)
(74) Vertreter: VOSSIUS & PARTNER
(86) Internationale Anmeldenummer: EP9903624
(87) Internationale Veröffentlichungsnummer: WO99063049

(56) Entgegenhaltungen:
- WO-A-91/01365
- PATENT ABSTRACTS OF JAPAN vol. 012, no. 275 (C-516), 29. Juli 1988 (1988-07-29) & JP 63 052870 A (FUJITSU LTD), 7. März 1988 (1988-03-07)
- PATENT ABSTRACTS OF JAPAN vol. 018, no. 648 (C-1284), 8. Dezember 1994 (1994-12-08) & JP 06 253812 A (MORITETSUKUSU:KK), 13. September 1994 (1994-09-13)

## Beschreibung

Die Erfindung betrifft ein Verfahren zum Transfer von beispielsweise in einem Muster angeordnetem biologischem Material, wobei man das biologische Material mit an einem Roboterkopf angebrachten Nadeln in Kontakt bringt und das biologische Material auf einen Träger transferiert, wobei die Nadeln mit einer biokompatiblen Schicht versehene Hartmetallnadeln sind. Die biokompatible Schicht setzt sich aus Metall-Stickstoffverbindungen zusammen. Ferner ist bevorzugt, daß die biokompatible Schicht mit einer korrosionsbeständigen Schicht unterlegt ist. Sofern das biologische Material in einem Muster angeordnet ist, sind die an dem Roboterkopf angebrachten Nadeln nach demselben Muster angeordnet. Es ist ferner bevorzugt, daß das Muster dem Muster der Anordnung der Vertiefungen in einer Mikrotiterplatte entspricht. Darüber hinaus betrifft die Erfindung einen Roboterkopf, der die erfindungsgemäßen Hartmetallnadeln aufweist. Insbesondere ist dieser Roboterkopf ein Bestandteil eines Pickingroboters und/oder eines Spottingroboters. Schließlich betrifft die Erfindung die Verwendung von mit einer biokompatiblen Schicht aus Metall-Stickstoffverbindungen versehenen Hartmetallnadeln zum Transfer von beispielsweise in einem Muster angeordnetem biologischem Material auf einen Träger.

Computer-assistierte Screening-Verfahren finden immer weiteren Eingang in biologisch oder biochemisch ausgerichtete Laboratorien. Wie exemplarisch im Human Genome Projekt erfahren, besteht ein Bedarf an Verfahren und Gerätschaften zum Nachweis und Katalogisieren von immer mehr Material in immer kürzeren Zeitabschnitten. Zum Screenen von Genbanken wurden in den letzten Jahren Roboter entwickelt, mit denen ein systematisches Absuchen der Banken und eine nachfolgende systematische Auswertung erheblich erleichtert wurde. Die in diesen Verfahren eingesetzten Roboter werden allgemein als Picking-/Spotting-Roboter bezeichnet. Die gegenwärtig eingesetzten Picking-/Spotting-Roboter sind in der Lage, biologisches Material aufzunehmen und gezielt abzulegen bzw. zu verteilen. Hierbei werden Gadgets (Nadelmatrizen) in verschiedenen Ausführungen verwendet, beispielsweise im Rasterformat 8 x 12 oder 16 x 24. Diese Nadelmatrizen sind mit Edelstahlnadeln ausgerüstet. Die Edelstahlnadeln haben eine gute Korrosionsbeständigkeit, jedoch eine geringe mechanische Festigkeit. Beim Einsatz der im Stand der Technik bekannten Picking-/Spotting-Roboter kommt es daher oft zu mechanischen Verformungen und so zu schlechten Trefferergebnissen (Picking) und damit zu zeitaufwendiger Nacharbeit. Beim Einsatz von Spottingnadeln in Spottingrobotern ergeben sich, beispielsweise auf Hochdichtefiltern, nach Verformung der Nadeln schlechte Raster biologischen Materials. JP-A-06 253 892 offenbart (implizit) ein Verfahren zum Transfer von biologischem Material. Das verwendete Gerät enthält eine "Fingerstation", die vermutlich mindestens eine Nadel umfaßt. Die charakterisierenden technischen Merkmale der Fingerstation sind nicht offenbart. WO-A-91/01365 offenbart ein Verfahren, in dem eine lagergezielte Entnahme und Zugabe der Proben eines mit einem Ausgußrohr versehenen automatischen Armes erreicht wird. Über die chemische Beschaffenheit des mit den Proben in Kontakt kommenden Materials ist nichts ausgesagt.

Die der vorliegenden Erfindung zugrundeliegende Aufgabe bestand somit darin, das aus dem Stand der Technik bekannte Verfahren so zu modifizieren, daß eine gezielte Ablage bzw. Verteilung von biologischem Material beim Einsatz von Picking-/Spotting-Robotern gewährleistet ist. Darüber hinaus soll selbstverständlich das biologische Material nach der Ablage bzw. der Verteilung seine biologischen Eigenschaften möglichst weitgehend beibehalten.

Diese Aufgabe wird durch die in den Ansprüchen dargestellten Ausführungsformen gelöst. Somit betrifft die Erfindung ein Verfahren zum Transfer von biologischem Material, wobei man das biologische Material mit an einem Roboterkopf angebrachten Nadeln in Kontakt bringt und das biologische Material auf einen Träger transferiert, dadurch gekennzeichnet, daß die Nadeln mit einer biokompatiblen Schicht aus Metall-Stickstoffverbindungen versehene Hartmetallnadeln sind.

Überraschenderweise wurde erfindungsgemäß gefunden, daß ein Verfahren gemäß dem vorstehend genannten Oberbegriff den gewünschten Effekt bringt, wenn die aus dem Stand der Technik bekannten Edelstahlstifte durch mit einer biokompatiblen Schicht versehene Hartmetallnadeln aus Metall-Stickstoffverbindungen ersetzt werden. Diese Maßnahme führt dazu, daß mit dem erfindungsgemäßen Verfahren insgesamt eine hohe Trefferquote bzw. langfristige Verwendung der eingesetzten Roboterköpfe gewährleistet ist. Die so behandelten Hartmetallnadeln haben sich als korrosionsbeständig erwiesen und eine minimierte Reibung im Gadget erzeugt. Sie zeichnen sich ferner durch hohe Abriebfestigkeit aus und sind verschleißfest. Die Beschichtung mit einer biokompatiblen Schicht hat eine bis zu 20-fach größere Haltbarkeit gegenüber einer üblichen Goldbeschichtung ergeben.

Mit dem erfindungsgemäßen Verfahren lassen sich hohe Treffergenauigkeiten beim Picken erzielen, wobei keine mechanische Verformung festgestellt wurde. Die im Stand der Technik bekannten Änderungen bzw. Verformungen an der Pickingnadel, welche aufgrund von Lageveränderungen im Gadget zu einer Verschlechterung der Treffergenauigkeit führten, fallen nunmehr weg. Damit ist auch das Problem einer computergesteuerten Korrektur hinfällig, die im Stand der Technik unzureichend gelöst war. Die bekannten Korrektursysteme korrigieren nämlich nur Lagefehler des gesamten Pickingkopfes. Eine Korrektur mittels Software war bei verbogenen Nadeln nicht möglich. Das erfindungsgemäße Verfahren erlaubt somit eine optimale Ausnutzung des Kamera-Korrektursystems, das vor allem Lagefehler des Pickingkopfes korrigiert. Durch die oben beschriebene verringerte Reibung kommen insgesamt weniger Störfälle durch klemmende Nadeln vor. Auch die Reinigung des Pickingkopfes ist unproblematisch und kann beispielsweise in Wasser durchgeführt werden. Schließlich läßt die höhere Stabilität/Haltbarkeit der Nadeln eine dichtere Anordnung auf dem Roboterkopf zu.

Im einer bevorzugten Ausführungsform des erfindungsgemäßen Verfahrens besteht die biokompatible Schicht aus TiN, TiCN, TiAlN oder CrN. Sofern Titannitrid als biokompatible Schicht verwendet wird, ist die Schichtdicke beispielsweise 4 µm bei 2400 HV. Das Titannitrid kann beispielsweise durch physikalische Abscheidung in der Dampfphase (PVD, Physical Vapor Deposition) aufgetragen werden. Dabei wird Titan mittels Lichtbogen verdampft und gleichzeitig Stickstoff im Hochvakuum zugeführt. Die Beschichtungstemperatur liegt dabei üblicherweise unter 500°C, wobei keine Gefügeumwandlungen und kein Wärmeverzug bzw. keine Wärmespannung auftreten. Durch die geringe Reaktivität von Titannitrid gegenüber Eisenwerkstoffen wird der Werkstoffverschleiß entscheidend herabgesetzt. Die Titancarbonnitrid-Schicht ist durch ihre höhere Schichthärte eine gute Ergänzung zur Titannitrid-Schicht. Sie weist üblicherweise eine Härte von 3000 HV bei einer Schichtdicke von ca. 3 µm auf. Die Titan-Aluminiumnitrid-Schicht wird durch die hohe Härte und Oxidationsbeständigkeit bei härtesten Einsatzbedingungen gewählt. Sie weist üblicherweise bei einer Schichtdicke bis ca. 3 mm eine Härte von 3300 HV auf. Die Verwendung einer Chromnitrid-Beschichtung erlaubt eine relativ hohe Härte in Verbindung mit einer geringen Sprödigkeit das Abscheiden auch dickerer Schichten. Die Härte dieser Art von beschichteten Nadeln beträgt bei einer Schichtdicke bis maximal 50 µm etwa 2000 HV.

Erfindungsgemäß werden auch Gemische der obengenannten biokompatiblen Schichten eingesetzt.

In einer weiteren bevorzugten Ausführungsform des erfindungsgemäßen Verfahrens sind die Hartmetallnadeln Auswerferstifte oder Schneidstempel. Die Nadeln oder Stempel können beispielsweise aus legiertem Kaltarbeitsstahl (WS), z.B. aus Material mit den Werkstoffnummern 1.2516, 1.2210 oder 1.2842 (z.B. DIN 1530/ISO 6751), sein. Die Härte des Nadelkopfes beträgt bei Verwendung von Mat.-Nr. WS 1.2516, üblicherweise 45 HRC ± 2 HRC, während der Nadelschaft der Nadelspitze eine Härte von üblicherweise 60 HRC ± 2 HRC aufweist. In dieser bevorzugten Ausführung sind diese Stifte gehärtet. Die Wärmebeständigkeit beträgt etwa 250°C. Nach Beschichtung mit beispielsweise Titannitrid weisen die Auswerferstifte eine Härte von etwa 2400 HV auf. Die vorgenannten Materialien weisen eine Anlaßbeständigkeit von mindestens 200°C auf. Es handelt sich dabei um zähharten Werkzeugstahl mit mittlerer Verschleißfestigkeit.

Weiterhin eingesetzt werden kann hochlegierter Werkzeugstahl (HWS), beispielsweise Materialien mit den Werkstoffnummern 1.2601 oder 1.2379. Diese Werkzeugstähle haben eine hohe Verschleißfestigkeit und hohe Anlaßbeständigkeit. Bei Einsatz eines Werkzeugstahls mit der Materialnummer WS 1.2379 weist die Nadelspitze/der Nadelschaft eine Härte von üblicherweise 62 ± 2 HRC auf. Der Nadelkopf weist üblicherweise eine Härte von 50 ± 5 HRC auf. Weiterhin kann hochlegierter Schnellarbeitsstahl (HSS), z.B. mit der Werkstoffnummer 1.3343, im erfindungsgemäßen Verfahren eingesetzt werden. Derartige Materialien zeichnen sich durch höchsten Verschleißwiderstand, gute Zähigkeit und hohe Wärmebeständigkeit aus. Der Einsatz eines derartigen Stahls, beispielsweise mit der Materialnummer WS 1.3343, ergibt üblicherweise für den Nadelschaft/die Nadelspitze eine Härte von 64 ± 2 HRC, wohingegen der Nadelkopf eine Härte von 50 ± 5 HRC aufweist. Ferner bevorzugt eingesetzt im erfindungsgemäßen Verfahren wird pulvermetallurgisch hergestellter Schnellarbeitsstahl (ASP 23). Derartige Materialien weisen eine ausgezeichnete Verschleißfestigkeit und Druckfestigkeit auf und zeichnen sich ferner durch hohe Zähigkeit sowie durch sehr gute Homogenität des Werkstoffes aus. Wenn beispielsweise ein derartiger Schnellarbeitsstahl mit der Materialnummer ASP 23 eingesetzt wird, hat der Nadelschaft/die Nadelspitze üblicherweise eine Härte von 64 ± 2 HRC. Der Nadelkopf weist üblicherweise eine Härte von 50 ± 5 HRC auf.

In einer weiteren bevorzugten Ausführungsform des erfindungsgemäßen Verfahrens ist der Roboterkopf der Kopf eines Pickingroboters.

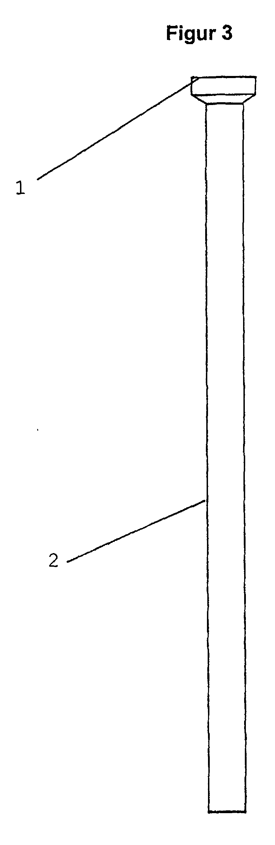

In einer anderen Ausführungsform des erfindungsgemäßen Verfahrens ist dieses dadurch gekennzeichnet, daß das biologische Material in einem Muster angeordnet ist, und man das biologische Material mit nach demselben Muster an einem Roboterkopf angebrachten Nadeln in Kontakt bringt. Dieses Verfahren wird bevorzugt eingesetzt, wenn der Roboter ein Spottingroboter ist. Sofern dieser Robotertyp zur Replikation des biologischen Materials zur Ablage in flüssigem Medium verwendet wird, werden die Nadeln als Repliziernadeln bezeichnet, vgl. Figur 3.

In einer weiteren bevorzugten Ausführungsform des erfindungsgemäßen Verfahrens ist der Roboterkopf somit der Kopf eines Spottingroboters. Die vorstehend gemachten Anmerkungen zu den Vorteilen des erfindungsgemäßen Verfahrens in der Ausführungsform, daß der Roboterkopf der Kopf eines Pickungroboters ist, gelten für den Fall, daß der Roboterkopf der Kopf eines Spottingroboters ist, in entsprechendem Maße.

In einer weiteren bevorzugten Ausführungsform des erfindungsgemäßen Verfahrens entspricht das Muster der Anordnung dem Muster der Vertiefungen einer Mikrotiterplatte oder dem Muster von in entsprechend regelmäßiger Anordnung gestalteten Reaktionsgefäßen.

In dieser Ausführungsform des erfindungsgemäßen Verfahrens können beispielsweise in Mikrotiterplatten gewachsene Clone, beispielsweise aus Säugerzellen, direkt auf ein Material übertragen werden, das im Screening-Verfahren eingesetzt werden kann.

In einer weiteren bevorzugten Ausführungsform des erfindungsgemäßen Verfahrens umfaßt das biologische Material Nucleinsäuren, (Poly)peptide oder transformierte Wirtsorganismen. In dieser Ausführungsform zeichnet es sich insbesondere als Vorteil aus, daß das zur Beschichtung der Hartmetallnadeln verwendete Material biokompatibel ist. Somit kann nämlich das biologische Material weiter analysiert werden, ohne daß Verluste durch Inaktivierung oder chemische Degradation zu befürchten sind. Im Falle transformierter Wirtsorganismen beispielsweise können diese nach Transfer in lebensfähigem Zustand wieder angezogen und so weiter untersucht werden. Nucleinsäuren können nach üblichen Screeningverfahren wie Hybridisierungen weiter untersucht werden. (Poly)peptide können, ohne verfahrensbedingten Modifikationen zu unterliegen, beispielsweise mit geeigneten Antikörpern weiter untersucht werden.

In einer besonders bevorzugten Ausführungsform des erfindungsgemäßen Verfahrens sind die transformierten Wirtsorganismen Hefen, vorzugsweise Pistoria- oder Saccharomyces-Zellen, Bakterien, vorzugsweise E. coli, Insektenzellen, vorzugsweise Spodoptera frugiperda-Zellen, Pilzzellen, vorzugsweise Aspergillus-Zellen, Pflanzenzellen oder Säugerzellen.

Eine andere bevorzugte Ausführungsform der Erfindung betrifft ein Verfahren, in dem der Träger ein flüssiger Träger oder ein fester Träger ist. Besonders bevorzugt ist dabei, daß der flüssige Träger ein Kulturmedium, ein Medium für die Lagerung von biologischem Material, ein Reaktionspuffer oder eine Färbelösung ist. Ein Beispiel für einen Reaktionspuffer ist ein Puffer, welcher in einer Polymerasekettenreaktion (PCR) eingesetzt wird. Neben der eigentlichen Pufferlösung, die vom Fachmann nach üblichen Verfahren zusammengestellt werden kann, kann der Puffer weitere Komponenten organischen oder anorganischen Ursprungs enthalten. In einer anderen bevorzugten Ausführungsform der Erfindung ist der feste Träger eine Nitrocellulosemembran, an die Nucleinsäuren oder (Poly)peptide gebunden werden können, eine Polyvinylidenfluoridmembran, an die (Poly)peptide gebunden werden können, oder ein Glasträger, vorzugsweise für das Auftragen von (Poly)peptiden oder Nucleinsäuren.

Eine andere bevorzugte Ausführungsform des erfindungsgemäßen Verfahrens ist dadurch gekennzeichnet, daß die biokompatible Schicht mit einer korrosionsbeständigen Schicht unterlegt ist. Üblicherweise wird die unterlegte Schicht durch eine erste Beschichtung aufgebracht, während die biokompatible Schicht nachfolgend auf die Nadeln aufgebracht wird. Die korrosionsbeständige Schicht hat insbesondere den Effekt, daß die Nadeln vor Durchrosten geschützt sind. Sie verleihen den Nadeln daher eine weiter verbesserte Haltbarkeit. Dies kommt insbesondere dann zum Tragen, wenn die biokompatible Schicht durch Aufdampfen aufgetragen wird. An den Aufhängungspunkten der Nadeln wird nämlich beim Aufdampfen keine biokompatible Schicht aufgetragen. An diesen Stellen sind bei Auftragen einer korrosionsbeständigen Schicht auf die Nadeln zusätzliche Maßnahmen gegen beispielsweise Durchrosten getroffen.

Vorzugsweise ist die korrosionsbeständige Schicht eine Nickelschicht oder eine Chromschicht. Eine Beschichtung insbesondere mit Nickel hat auch den Vorteil, daß diese Schicht recht kostengünstig aufgebracht werden kann.

Die Erfindung betrifft auch einen Roboterkopf, insbesondere zur Durchführung des erfindungsgemäßen Verfahrens, der die vorstehend dargestellten, mit einer biokompatiblen Schicht aus Metall-Stickstoffverbindungen versehenen Hartmetallnadeln aufweist. Bevorzugt ist insbesondere, daß der Roboterkopf Bestandteil eines Pickingroboters oder eines Spottingroboters ist.

Schließlich betrifft die Erfindung die Verwendung der vorstehend dargestellten beschichteten Hartmetallnadeln zum Transfer von in einem Muster angeordnetem biologischem Material auf einem Träger.

Die Figuren zeigen:

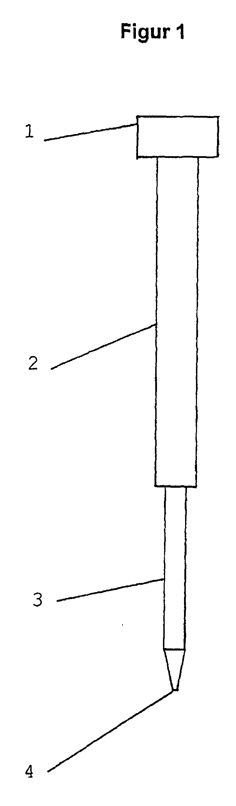

- **Figur 1**: zeigt eine Ausführungsform einer Spotting-Nadel mit Nadelkopf 1, Nadelschaft 2, abgesetztem Nadelschaft 3 und Nadelspitze 4. Der Nadelkopf weist in dieser Ausführungsform eine Länge von 2,0 mm und einen Durchmesser von 4,0 mm auf. Die Maße für den Nadelschaft sind: Länge: 16,0 mm, Durchmesser: 2,0 mm. Der abgesetzte Nadelschaft weist eine Länge von 8,0 mm und einen Durchmesser von 1,0 mm auf. Die Nadelspitze schließlich weist eine Länge von 2,0 mm und einen Durchmesser von 250 µm bzw. 400 µm auf.

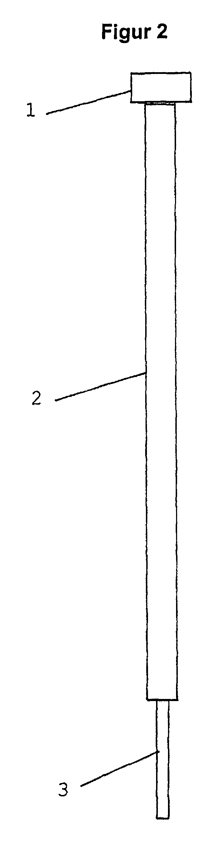

- **Figur 2**: zeigt eine Ausführungsform einer Picking-Nadel, in der Nadelkopf 1, Nadelschaft 2 und Nadelspitze 3 erkennbar sind. Der Nadelkopf weist in dieser Ausführungsform eine Länge von 2,0 mm und einen Durchmesser von 4,0 mm auf. Der Nadelschaft hat eine Länge von 40,0 mm und einen Durchmesser von 2,0 mm. Die Nadelspitze schließlich weist eine Länge von 8,0 mm und einen Durchmesser von 0,8 mm auf.
- **Figur 3**: zeigt eine Ausführungsform einer Replizier-Nadel mit einem Nadelkopf 1 und einem Nadelschaft 2. Der Nadelkopf weist eine Länge von 1,0 mm und einen Durchmesser von 2,0 mm auf, wohingegen der Nadelschaft eine Länge von 29,0 mm und einen Durchmesser von 1,2 mm aufweist.

Die Beispiele erläutern die Erfindung.

### Beispiel 1

### Einsatz von Hartmetallnadeln mit Titanbeschichtung beim Spotting

Transformierte CHO-Zellen werden aus Mikrotiterplatten (384 Vertiefungen) mittelns Gadget entnommen und in einem hochauflösenden Raster auf Nylonmembranen übertragen. Die Filter werden für eine Hybridisierung nach üblichen Verfahren vorbereitet und mit einer für das in die CHO-Zellen eingeschleuste Fremdgen spezifischen Sonde, die eine radioaktive Markierung trägt, hybridisiert. Nach dem Waschen der Filter erfolgt eine Auswertung mittels computergesteuerter Bildanalyse. Dabei werden die positiven Ereignisse (Hybridisierungen) in dem vordefinierten Raster erwartet. Die mit dem im Stand der Technik bekannten Spotting-Nadeln (Figur 1) durchgeführten Versuche weisen eine höhere Anzahl von Rasterfehlern auf als die Versuche, welche mit den vorstehend beschriebenen, im erfindungsgemäßen Verfahren eingesetzten Nadeln durchgeführt wurden. Die Ergebnisse zeigen, daß die automatische Auswertung erheblich erschwert wird bzw. unmöglich gemacht wird, wenn Spotting-Nadeln aus im Stand der Technik bekanntem Material eingesetzt werden.

### Beispiel 2

### Einsatz von Hartmetallnadeln mit Titanbeschichtung beim Picking

Es wurden Roboterköpfe verglichen, die mit Nadeln bestückt waren, welche aus aus dem Stand der Technik bekannten Materialien bestanden bzw. bei denen die Nadeln Auswerferstifte waren, welche mit Titannitrid beschichtet waren. Beim Picking werden im Kulturmedium gewachsene transformierte Kolonien (CHO-Zellen) mit Hilfe eines Kamerasystems erkannt und anschließend durch Picking-Nadeln (Figur 2) aufgenommen, um sie in Mikrotiterplatten sortiert abzulegen. Die im Stand der Technik bekannten Nadeln führten hierbei zu schlechteren Trefferergebnissen. Beispielsweise wurden die Kolonien nur gestreift und somit weniger Material übertragen. Mit im Stand der Technik bekannten Nadeln wurden in einzelnen Fällen Kolonien auch ganz verfehlt, was ebenfalls zu einer suboptimalen Ergebnisausbeute führt. Mit den im Zusammenhang dieser Erfindung beschriebenen Picking-Nadeln wurden hingegen über einen längeren Zeitraum (4 Monate) keine Verformungen und damit keine Fehler beim sortierten Ablegen festgestellt.

### Beispiel 3

### Einsatz von Hartmetallnadeln mit Titanbeschichtung beim Spotting von PCR-Produkten

Die durch eine Polymerasekettenreaktion (PCR) unter Verwendung von vektorspezifischen Primern nach Standardverfahren in einer Mikrotiterplatte (384 Vertiefungen) synthetisierten Nucleinsäuren einer Pankreas-cDNA-Genbank werden mittels Gadget entnommen und in einer möglichst dichten Anordnung (s. u.) auf einen Glasträger ausgebracht. Durch Hybridisierung mit geeigneten insulinspezifischen Sonden, die durch nichtradioaktive Verfahren (fluoreszierende Farbstoffe) nach Standardverfahren markiert wurden, konnte die Expression des durch ein PCR-Produkt repräsentierten Insulin-Gens in Pankreas-Gewebe ermittelt und quantifiziert werden. Die Auswertung dieser sogenannten Mikroarrays erfolgte durch eine computerunterstützte Bildanalyse. Hierzu war eine reproduzierbare, möglichst exakte Einhaltung des Spottingmusters erforderlich, die erst durch die in dieser Erfindung beschriebenen Nadeln erreicht werden kann.

Um eine möglichst große Zahl von Genen gleichzeitig auswerten zu können, ist eine möglichst dichte Anordnung der PCR-Produkte auf dem Glasträger wünschenswert. Werden die PCR-Produkte durch Spotting ausgebracht, so ist diese dichte Anordnung durch die Dicke der Nadeln limitiert. Diese kann durch die erfindungsgemäß beschriebene Prozedur der Versteifung der Spottingnadeln weiter herabgesetzt werden.

Bei einem Durchmesser der Nadelspitze von 50 µm konnten so die Spots in einem Abstand von 50 µm angeordnet werden. Bei diesen geringen Abständen führen kleinste Verformungen, wie im Stand der Technik zu beobachten, zu schwierig auswertbaren bzw. nicht auswertbaren Rastern.

Die in dieser Erfindung beschriebenen Nadeln ermöglichen somit das routinemäßige Ausbringen von PCR-Produkten in größeren Chargen auf Glasträgern durch Spotting in einer im Zuge moderner Detektionsverfahren geforderten Dichte und Exaktheit.

## Patentansprüche

1. Verfahren zum Transfer von biologischem Material, wobei man das biologische Material mit an einem Roboterkopf angebrachten Nadeln in Kontakt bringt und das biologische Material auf einen Träger transferiert, **gekennzeichnet durch** das Bereitstellen von Hartmetallnadeln, die mit einer biokompatiblen Schicht aus Metall-Stickstoffverbindungen versehen ist.

2. Verfahren nach Anspruch 1, **dadurch gekennzeichnet, daß** die biokompatible Schicht aus Titannitrid, TiCN, TiAlN oder CrN oder einem Gemisch daraus besteht.

3. Verfahren nach Anspruch 1 oder 2, **dadurch gekennzeichnet, daß** die Hartmetallnadeln Auswerferstifte oder Schneidstempel sind.

4. Verfahren nach einem der Ansprüche 1 bis 3, **dadurch gekennzeichnet, daß** der Roboterkopf der Kopf eines Pickingroboters ist.

5. Verfahren nach einem der Ansprüche 1 bis 3, **dadurch gekennzeichnet, daß** das biologische Material in einem Muster angeordnet ist und man das biologische Material mit nach demselben Muster an einem Roboterkopf angebrachten Nadeln in Kontakt bringt.

6. Verfahren nach einem der Ansprüche 1 bis 3 und 5, **dadurch gekennzeichnet, daß** der Roboterkopf der Kopf eines Spottingroboters ist.

7. Verfahren nach Anspruch 5 oder 6, **dadurch gekennzeichnet, daß** das Muster der Anordnung der Vertiefungen einer Mikrotiterplatte oder dem Muster von in entsprechend regelmäßiger Anordnung gestalteten Reaktionsgefäßen entspricht.

8. Verfahren nach einem der Ansprüche 1 bis 7, **dadurch gekennzeichnet, daß** das biologische Material Nucleinsäuren, (Poly)peptide oder transformierte Wirtsorganismen umfaßt.

9. Verfahren nach Anspruch 8, **dadurch gekennzeichnet, daß** die transformierten Wirtsorganismen Hefen, Bakterien, Insektenzellen, Pilzzellen, Pflanzenzellen oder Säugerzellen sind.

10. Verfahren nach Anspruch 9, wobei die Hefen Pistoria oder Saccharomyces-Zellen sind.

11. Verfahren nach Anspruch 9, wobei die Bakterien E. coli-Zellen sind.

12. Verfahren nach Anspruch 9, wobei die Insektenzellen Spodoptera frugiperda-Zellen sind.

13. Verfahren, nach Anspruch 9, wobei die Pilzzellen Aspergillus-Zellen sind.

14. Verfahren nach einem der Ansprüche 1 bis 13, **dadurch gekennzeichnet, daß** der Träger ein flüssiger Träger oder ein fester Träger ist.

15. Verfahren nach Anspruch 14, **dadurch gekennzeichnet, daß** der flüssige Träger ein Kulturmedium, ein Medium für die Lagerung von biologischem Material, ein Reaktionspuffer oder eine Färbelösung ist.

16. Verfahren nach Anspruch 15, **dadurch gekennzeichnet, daß** der feste Träger eine Nitrocellulosemembran, eine Poly-Vinylidenfluoridmembran oder ein Glasträger ist.

17. Verfahren nach einem der Ansprüche 1 bis 16, **dadurch gekennzeichnet, daß** die biokompatible Schicht mit einer korrosionsbeständigen Schicht unterlegt ist.

18. Verfahren nach Anspruch 17, **dadurch gekennzeichnet, daß** die korrosionsbeständige Schicht eine Nickelschicht oder eine Chromschicht ist.

19. Roboterkopf zur Durchführung eines Verfahrens nach einem der vorstehenden Ansprüche, **dadurch gekennzeichnet, daß** er Hartmetallnadeln mit einer biokompatiblen Schicht aus Metall-Stickstoffverbindungen aufweist.

20. Roboterkopf nach Anspruch 19, **dadurch gekennzeichnet, daß** er Bestandteil eines Pickingroboters oder eines Spottingroboters ist.

21. Verwendung von Hartmetallnadeln mit einer biokompatiblen Schicht, wie in einem der vorstehenden Ansprüche dargestellt, zum Transfer von biologischem Material auf einen Träger.

22. Verwendung nach Anspruch 21, wobei das biologische Material in einem Muster angeordnet ist.

## Claims

1. A method for transferring biological material, wherein the biological material is contacted with needles fixed to a robot head and the biological material is transferred onto a carrier, the method being **characterised by** the provision of hard metal needles having a biocompatible layer of metal nitrogen compounds.

2. The method according to claim 1, **characterised in that** the biocompatible layer consists of titanium nitride, TiCN, TiAlN or CrN or a mixture thereof.

3. The method according to claim 1 or 2, **characterised in that** the hard metal needles are ejector pins or clipping punches.

4. The method according to any one of claims 1 to 3, **characterised in that** the robot head is the head of a picking robot.

5. The method according to any one of claims 1 to 3, **characterised in that** the biological material is arranged in a pattern and the biological material is contacted with the needles fixed to a robot head in the same pattern.

6. The method according to any one of claims 1 to 3 and 5, **characterised in that** the robot head is the head of a spotting robot.

7. The method according to claim 5 or 6, **characterised in that** the pattern corresponds to the arrangement of the wells of a microtiter plate or to the pattern of reaction vessels having the corresponding uniform arrangement.

8. The method according to any one of claims 1 to 7, **characterised in that** the biological material comprises nucleic acids, (poly)peptides or transformed host organisms.

9. The method according to claim 8, **characterised in that** the transformed host organisms are yeasts, bacteria, insect cells, fungal cells, plant cells or mammalian cells.

10. The method according to claim 9, wherein the yeasts are Pistoria or Saccharomyces cells.

11. The method according to claim 9, wherein the bacteria are E. coli cells.

12. The method according to claim 9, wherein the insect cells are Spodoptera frugiperda cells.

13. The method according to claim 9, wherein the fungal cells are Aspergillus cells.

14. The method according to any one of claims 1 to 13 **characterised in that** the carrier is a liquid or a solid carrier.

15. The method according to claim 14 **characterised in that** the liquid carrier is a culture medium, a medium for storing biological material, a reaction buffer or a dye solution.

16. The method according to claim 15 **characterised in that** the solid carrier is a nitrocellulose membrane, a polyvinylidene fluoride membrane or a glass carrier.

17. The method according to any one of claims 1 to 16 **characterised in that** the biocompatible layer is based on a corrosion-proof layer.

18. The method according to claim 17 **characterised in that** the corrosion-proof layer is a nickel layer or a chromium layer.

19. A robot head for carrying out a method according to any one of the preceding claims **characterised in that** it has hard metal needles having a biocompatible layer of metal nitrogen compounds.

20. The robot head according to claim 19 **characterised in that** it forms part of a picking robot or a spotting robot.

21. Use of hard metal needles having a biocompatible layer as explained in any of the preceding claims for transferring biological material onto a carrier.

22. Use according to claim 21, wherein the biological material is arranged in a pattern.

## Revendications

1. Procédé pour le transfert de matériau biologique, où l'on met en contact le matériau biologique avec des aiguilles fixées sur une tête de robot et où le matériau biologique est transféré sur un support, **caractérisé par** la disposition d'aiguilles en métal dur, qui sont munies d'une couche biocompatible en composés métal-azote.

2. Procédé selon la revendication 1, **caractérisé en ce que** la couche biocompatible se compose en du nitrure de titane, du TiCN, du TiAlN ou du CrN ou un mélange de ceux-ci.

3. Procédé selon la revendication 1 ou 2, **caractérisé en ce que** les aiguilles en métal dur sont des tiges d'éjection ou des poinçons d'ébavurage.

4. Procédé selon l'une quelconque des revendications 1 à 3, **caractérisé en ce que** la tête de robot est la tête d'un robot de prélèvement.

5. Procédé selon l'une quelconque des revendications 1 à 3, **caractérisé en ce que** le matériau biologique est disposé selon un modèle et **en ce que** l'on met en contact le matériau biologique avec des aiguilles fixées sur un tête de robot selon le même modèle.

6. Procédé selon l'une quelconque des revendications 1 à 3 et 5, **caractérisé en ce que** la tête de robot est la tête d'un robot applicateur.

7. Procédé selon la revendication 5 ou 6, **caractérisé en ce que** le modèle correspond à l'arrangement des puits d'une plaque de microtitrage ou au modèle des flacons de réaction installées suivant un arrangement régulier approprié.

8. Procédé selon l'une quelconque des revendications 1 à 7, **caractérisé en ce que** le matériau biologique comprend des acides nucléiques, des (poly)peptides ou des organismes hôtes transformés.

9. Procédé selon la revendication 8, **caractérisé en ce que** les organismes hôtes transformés sont des levures, des bactéries, des cellules d'insectes, des cellules de champignon, des cellules végétales ou des cellules de mammifères.

10. Procédé selon la revendication 9, où les levures sont des cellules de Pistoria ou de Saccharomycètes.

11. Procédé selon la revendication 9, où les bactéries sont des cellules de *E. coli*.

12. Procédé selon la revendication 9, où les cellules d'insectes sont des cellules de *Spodoptera frugiperda*.

13. Procédé selon la revendication 9, où les cellules de champignon sont des cellules de Aspergillus.

14. Procédé selon l'une quelconque des revendications 1 à 13, **caractérisé en ce que** le support est un support liquide ou un support solide.

15. Procédé selon la revendication 14, **caractérisé en ce que** le support liquide est un milieu de culture, un milieu pour le stockage de matériau biologique, un tampon de réaction ou une solution de colorant.

16. Procédé selon la revendication 15, **caractérisé en ce que** le support solide est une membrane en nitrocellulose, une membrane en poly fluorure de vinylidène ou un support en verre.

17. Procédé selon l'une quelconque des revendications 1 à 16, **caractérisé en ce que** la couche biocompatible est doublée d'une couche résistant à la corrosion.

18. Procédé selon la revendication 17, **caractérisé en ce que** la couche résistant à la corrosion est une couche en nickel ou une couche en chrome.

19. Tête de robot pour réaliser un procédé selon l'une quelconque des revendications précédentes, **caractérisée en ce qu'**elle présente des aiguilles en métal dur avec une couche biocompatible en des composés métal-azote.

20. Tête de robot selon la revendication 19, **caractérisée en ce qu'**elle est un constituant d'un robot de prélèvement ou d'un robot d'application.

21. Utilisation d'aiguilles en métal dur avec une couche biocompatible, comme indiqué dans l'une quelconque des revendications précédentes, pour le transfert de matériau biologique sur un support.

22. Utilisation selon la revendication 21, où le matériau biologique est disposé selon un modèle.
